(19) Europäisches Patentamt
European Patent Office
Office européen des brevets

(11) Veröffentlichungsnummer: **0 289 782 B1**

(12) **EUROPÄISCHE PATENTSCHRIFT**

(45) Veröffentlichungstag der Patentschrift: **04.03.92**

(51) Int. Cl.$^5$: **A61K 7/13**, C07C 215/00, C07C 215/10

(21) Anmeldenummer: **88105126.2**

(22) Anmeldetag: **29.03.88**

(54) **Verwendung von 2,6-Dinitro-anilinderivaten in Haarfärbemitteln und neue 2,6-Dinitro-anilinderivate.**

(30) Priorität: **04.05.87 DE 3714775**

(43) Veröffentlichungstag der Anmeldung:
**09.11.88 Patentblatt 88/45**

(45) Bekanntmachung des Hinweises auf die Patenterteilung:
**04.03.92 Patentblatt 92/10**

(84) Benannte Vertragsstaaten:
**AT BE CH DE ES FR GB GR IT LI NL SE**

(56) Entgegenhaltungen:
**BG-A- 1 116 138**
**GB-A- 2 090 853**
**US-A- 3 654 363**
**US-A- 4 395 577**
**FR-A- 1 511 025**
**GB-A- 2 168 369**
**US-A- 3 699 167**

(73) Patentinhaber: **Wella Aktiengesellschaft**
**Berliner Allee 65**
**W-6100 Darmstadt(DE)**

(72) Erfinder: **Clausen, Thomas, Dr.**
**Ernst Pasqué Strasse 35 A**
**W-6146 Alsbach(DE)**
Erfinder: **Konrad, Eugen**
**Mecklenburger Strasse 101**
**W-6100 Darmstadt(DE)**

**EP 0 289 782 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist die Verwendung von Nitrofarbstoffen zum Färben von Haaren, wobei als Nitrofarbstoffe bestimmte 2,6-Dinitro-anilinderivate verwendet werden, sowie Mittel mit einem Gehalt an 2,6-Dinitro-anilinderivaten und neue 2,6-Dinitro-anilinderivate.

Nitrofarbstoffe finden heute in Haarfärbemitteln eine breite Anwendung. Sie werden in Oxidationshaarfärbemitteln als Zusätze für die Erzeugung von natürlichen oder modischen Farbnuancen verwendet. Durch Kombination von mehreren verschieden gefärbten Nitrofarbstoffen ist es jedoch auch möglich, Haarfärbemittel herzustellen, die Haare in natürlichen bis modischen Nuancen ohne die Anwendung von Oxidationsmitteln zu färben vermögen.

So können zum Beispiel durch die Kombination eines orangefärbenden mit einem blaufärbenden Nitrofarbstoff natürlich wirkende, braune Färbungen erzeugt werden. Daneben ist es jedoch auch möglich, mit einem gelbfärbenden und einem violettfärbenden Nitrofarbstoff ein ähnliches Ergebnis zu erhalten. Es werden daher gelbe Nitrofarbstoffe benötigt, die das Haar entweder in einem intensiven, reinen Zitronengelb zu färben vermögen, welches möglichst frei von Rotanteilen sein muß, oder solche, die orange färben und in Kombination mit rein blauen Farbstoffen eingesetzt werden können.

Daneben werden an Haarfarbstoffe noch viele weitere Anforderungen gestellt. Die Nitrofarbstoffe müssen in toxikologischer und dermatologischer Hinsicht unbedenklich sein. Ihre Verwendung in Oxidationshaarfärbemitteln setzt voraus, daß sie in Anwesenheit von Wasserstoffperoxid in alkalischer Lösung stabil sind. Außerdem wird für die erzeugten Haarfärbungen eine gute Licht-, Säure- und Reibechtheit gefordert. Schließlich sollten die Nitroverbindungen durch möglichst einfache Verfahren herstellbar sein.

Die bisher in der Literatur zur Gelbfärbung des Haares beschriebenen substituierten Amino-nitrophenole erfüllen die vorstehend genannten Voraussetzungen nur unzureichend. Das im International Journal of Cosmetic Science 1982, Seiten 25 - 35 genannte 4-Nitro-3-(2′-hydroxyethyl)amino-phenol ergibt zwar eine zitronengelbe Färbung, diese ist jedoch sehr farbschwach. Zwei weitere Isomere, nämlich das 4-Nitro- und das 5-Nitro-2-(2′-hydroxyethyl)amino-phenol, sind aufgrund der aromatischen Hydroxygruppen pH-empfindlich und zeigen unerwünschte Farbänderungen bei Säure- oder Alkalieinwirkung.

Weitere bekannte gelbe Nitrofarbstoffe sind die in der DE-AS 1 619 395 genannten o-, m- und p-Nitroanilinderivate. Diese Verbindungen erfüllen zwar weitgehend die Anforderungen in anwendungstechnischer Hinsicht, befriedigen aber nicht im Hinblick auf die physiologischen Eigenschaften.

In der GB-A-2 090 853 sind bestimmte 2-Amino-6-nitro-anilinderivate als Haarfarbstoffe beschrieben. Diese färben jedoch Haare in farbschwachen, wenig brillianten Tönen.

Die in der GB-A-2 168 369 beschriebenen 2,4-Dinitro-anilinderivate färben zwar gelb, jedoch relativ farbschwach und sind infolge des speziellen mehrstufigen Herstellungsverfahrens auf bestimmte Stellungsisomere beschränkt.

In der US-A-3 654 363 sind bestimmte 2,6-Dinitro-p-phenylendiaminderivate als Haarfarbstoffe beschrieben. Jene Verbindungen färben Haare jedoch rot-violett.

Die in der DE-OS 3 442 757 beschriebenen 2-Nitro-anilinderivate erfüllen zwar weitgehend die gestellten Anforderungen, jedoch läßt sich die durch Variation der Substituenten erzielbare Farbskala nicht bis in den Rotbereich hin ausdehnen. Deshalb können mit den dort beschriebenen 2-Nitro-anilinderivaten ohne die zusätzliche Anwendung von Rotfarbstoffen keine modischen rötlichen Haarfarben hergestellt werden.

Es bestand demnach die Aufgabe, Nitroanilinderivate für die Verwendung in Haarfärbemitteln zur Verfügung zu stellen, welche die vorteilhaften Eigenschaften der Verbindungen gemäß der DE-OS 3 442 757 aufweisen und darüberhinaus eine Ausweitung der Farbskala (durch Variation der Substituenten) in den Rotbereich ermöglichen.

Es wurde nun gefunden, daß durch die Verwendung eines 2,6-Dinitro-anilinderivates der allgemeinen Formel (I)

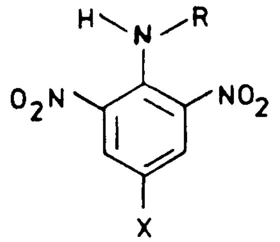

(I),

wobei R die Bedeutung H, $C_1$-$C_4$-Alkyl, $C_2$-$C_4$-Monohydroxyalkyl oder $C_3$-$C_4$-Dihydroxyalkyl hat und X einen der Reste $C_1$-$C_4$-Alkyl, $C_2$-$C_4$-Monohydroxyalkyl, $C_1$-$C_4$-Perfluoralkyl,$C_1$-$C_4$-Alkoxy, $C_2$-$C_4$-Monohydroxyalkoxy, $C_3$-$C_4$-Dihydroxyalkoxy oder Halogen darstellt, als Farbstoff in Haarfärbemitteln die gestellte Aufgabe in hervorragender Weise gelöst wird.

Durch Variation des Restes X stehen Verbindungen der Formel (I) zur Verfügung, welche nunmehr alle benötigten Farbtöne, von einem blaustichigen Zitronengelb über ein reines Gelb und Orange bis zu einem Rotorange liefern.

Im Vergleich zu den Farbstoffen des 2-Nitro-anilintyps gemäß der DE-OS 3 442 757 läßt sich mit den Verbindungen der allgemeinen Formel (I) die Farbskala bis in den Rotbereich hinein ausdehnen. Hierdurch ist es möglich, durch Kombination dieser Farbstoffe mit geeigneten blauen oder violetten Farbstoffen nicht nur natürlich wirkende, braune Färbungen zu erzeugen, sondern ohne die zusätzliche Anwendung von Rotfarbstoffen auch modische, rötliche Haarfarben herzustellen.

Von den unter die Formel (I) fallenden Verbindungen ist aus färberischen und physiologischen Gründen die Verwendung von 2,6-Dinitro-anilinderivaten der allgemeinen Formel (I), wobei R die Bedeutung 2-Hydroxyethyl oder 2,3-Dihydroxy-propyl hat und X einen der Reste $CH_3$, $CF_3$, $CH_2OH$, $OCH_3$, $OCH_2CH_2OH$, $OCH_2CH(OH)CH_2OH$, Cl oder Br darstellt, als Farbstoff in Haarfärbemitteln bevorzugt.

Beispiele für erfindungsgemäß geeignete 2,6-Dinitro anilinderivate der allgemeinen Formel (I) sind
2,6-Dinitro-4-methyl-(2′-hydroxyethyl)-anilin,
2,6-Dinitro-4-methyl-(2′,3′-dihydroxypropyl)-anilin,
2,6-Dinitro-4-trifluormethyl-(2′,3′-dihydroxypropyl)-anilin,
2,6-Dinitro-4-trifluormethyl-(2′-hydroxyethyl)-anilin,
2,6-Dinitro-4-methoxy-(2′-hydroxyethyl)-anilin,
2,6-Dinitro-4-methoxy-(2′,3′-dihydroxypropyl)-anilin,
2,6-Dinitro-4-(2′-hydroxyethyloxy)-(2″,3″-dihydroxypropyl)-anilin,
2,6-Dinitro-4-(2′-hydroxyethyloxy)-(2″-hydroxyethyl)-anilin und
4-Chlor-2,6-dinitro-(2′,3′-dihydroxypropyl)-anilin.

Überraschend sind die sehr guten toxikologischen und physiologischen Eigenschaften der Verbindungen der allgemeinen Formel (I). So sind alle getesteten Farbstoffe, beispielsweise diejenigen, bei denen R eine Hydroxyethylgruppe oder eine Dihydroxypropylgruppe bedeutet und X einen der Reste Methyl, Trifluormethyl oder Methoxy darstellt, nicht mutagen und werden daher und aus Gründen der Löslichkeit und der Farbtiefe auch gegenüber den nicht hydroxyalkylierten Nitroanilinderivaten besonders bevorzugt.

Die Verbindungen der allgemeinen Formel (I) stellen für die Färbung von menschlichen Haaren hervorragend geeignete, gelb bis rotorange färbende Nitrofarbstoffe dar. Sie sind gut in Wasser löslich und weisen eine ausgezeichnete Lagerstabilität auf.

Gegenstand der vorliegenden Anmeldung ist daher ebenfalls ein Mittel zur Färbung von Haaren mit einem Farbstoffgehalt und für Haarfärbemittel üblichen kosmetischen Zusätzen, dadurch gekennzeichnet, daß es ein 2,6-Dinitro-anilinderivat der allgemeinen Formel (I), vorzugsweise in einer Menge von 0,01 bis 2,0 Gewichtsprozent, enthält. Von diesem Mittel ist jenes bevorzugt, welches ein 2,6-Dinitro-anilinderivat der Formel (I) enthält, worin R 2-Hydroxyethyl oder 2,3-Dihydroxypropyl darstellt und X die in der Formel (I) angegebene Bedeutung hat.

Das erfindungsgemäße Mittel zur Färbung von Haaren betrifft sowohl eine Ausführungsform, bei der es ohne Zugabe eines Oxidationsmittels angewendet wird, als auch eine weitere Ausführungsform bei der die Zugabe eines Oxidationsmittels erforderlich ist.

Bei dem ersteren Haarfärbemittel ohne Oxidationsmittel-Zugabe handelt es sich um ein Mittel, welches neben den Farbstoffen der angegebenen Formel (I) noch andere bekannte direkt auf das Haar aufziehende Farbstoffe enthalten kann. Von diesen für die Haarfärbung bekannten Farbstoffen seien beispielsweise die folgenden erwähnt: Aromatische Nitrofarbstoffe, wie zum Beispiel 2-Amino-4-nitro-phenol, Pikraminsäure, 1-(2′-Hydroxy ethyl)amino-2-amino-4-nitro-benzol, 4-(2′-Ureidoethyl)amino-nitrobenzol, 4-(2′,3′-Dihydroxypropyl)amino-3-nitro-trifluormethylbenzol, 4-(2′-Hydroxyethyl)amino-3-nitro-toluol, 1,4-Bis(2′-hydroxyethyl)-amino-4-N-ethyl-2-nitro-benzol, 2 Nitro-4-(2′-hydroxyethyl)amino-anilin, 4-Bis(2′-hydroxyethyl)-amino-1-methylamino-2-nitro-benzol, 2,5-Bis(2′-hydroxyethyl)amino-nitrobenzol, 2-(2′-Hydroxyethyl)amino-4,6-dinitrophenol, 1-Amino-4-(2′,3′-dihydroxypropyl)amino-2-nitro-5-chlor-benzol, 4-Bis(2′-hydroxyethyl)amino-1-methylamino-2-nitro-benzol und 1-Amino-2-nitro-4-bis(2′-hydroxyethyl)amino-benzol; Triphenylmethanfarbstoffe, wie zum Beispiel Basic Violet 1 (C.I. 42 535); Azofarbstoffe, wie zum Beispiel Acid Brown 4 (C.I 14 805); Anthrachinonfarbstoffe, wie zum Beispiel Disperse Blue 23 (C. I. 61 545), Disperse Violet 4 (C.I. 61 105), 1,4,5,8-Tetraamino-anthrachinon und 1,4-Diamino-anthrachinon, wobei die Farbstoffe dieser Klassen je nach Art ihrer Substituenten sauren, nicht-ionogenen oder basischen Charakter haben können. Weitere geeignete, direkt auf das Haar aufziehende Farbstoffe sind beispielsweise in dem Buch von J.C. Johnson, "Hair Dyes" Noyes Data Corp. Park-Ridge (USA) (1973), Seiten 3 - 91 und 113 - 139 (ISBN: 0-8155-0477-2) beschrieben.

Die Zubereitungsform des hier beschriebenen Haarfärbemittels auf der Basis von direkt auf das Haar aufziehenden Farbstoffen kann beispielsweise eine Lösung, insbesondere eine wäßrige oder wäßrig-alkoholische Lösung, sein. Bevorzugte Zubereitungsformen sind weiterhin eine Creme, ein Gel oder eine Emulsion, wobei sie auch im Gemisch mit einem Treibgas oder mittels einer Pumpe versprüht werden können.

Die Farbstoffe der allgemeinen Formel (I) sollen in diesem Haarfärbemittel ohne Oxidationsmittel-Zugabe in einer Konzentration von etwa 0,01 bis 2,0 Gewichtsprozent, vorzugsweise von 0,01 bis 1,0 Gewichtsprozent, enthalten sein Der Gesamtgehalt an den direkt auf das Haar aufziehenden Farbstoffen liegt in den Grenzen von etwa 0,01 bis 3,0 Gewichtsprozent.

Der pH-Wert dieses Färbemittels liegt im Bereich von 3 bis 10,5, insbesondere bei pH 7,5 bis 9,5, wobei die Einstellung des gewünschten alkalischen pH-Wertes hauptsächlich mit Ammoniak erfolgt, jedoch auch mit organischen Aminen wie beispielsweise Monoethanolamin oder Triethanolamin vorgenommen werden kann.

Seine Anwendung erfolgt in üblicher Weise durch Aufbringen einer für die Haarfärbung ausreichenden Menge des Mittels auf die Haare, mit denen es eine Zeitlang, etwa 5 bis 30 Minuten, in Berührung bleibt. Anschließend wird mit Wasser, gegebenenfalls noch mit der wäßrigen Lösung einer schwachen organischen Säure, gespült und getrocknet. Als schwache organische Säure können beispielsweise Essigsäure, Zitronensäure, Weinsäure und ähnliche Verwendung finden.

Das vorstehend beschriebene Haarfärbemittel ohne Oxidationsmittel-Zugabe kann selbstverständlich auch kosmetische Polymerisate enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbindungen wie Acrylsäure- beziehungsweise Methacrylsäurepolymerisate, basische Polymerisate von Estern aus diesen beiden Säuren und Aminoalkoholen beziehungsweise deren Salze oder Quaternierungsprodukte, Polyacrylnitril, Polyvinyllactame sowie Copolymerisate aus derartigen Verbindungen wie zum Beispiel Polyvinylpyrrolidon-Vinylacetat erwähnt.

Auch natürliche Polymere, wie Chitosan (entacetyliertes Chitin) oder Chitosanderivate, können für den genannten Zweck eingesetzt werden.

Die Polymerisate sind in diesem Mittel in der für solche Mittel üblichen Menge von etwa 1 bis 5 Gewichtsprozent enthalten. Der pH-Wert des Mittels liegt im Bereich von etwa 6,0 bis 9,0.

Die Anwendung dieses Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich kann das vorstehend beschriebene Haarfärbemittel ohne Oxidationsmittel-Zugabe gegebenenfalls weitere, für Haarfärbemittel übliche Zusätze, wie zum Beispiel Pflegestoffe, Netzmittel, Verdicker, Weichmacher, Konservierungsstoffe und Parfümöle sowie auch andere, nachstehend für Oxidationshaarfärbemittel aufgeführte übliche Zusätze enthalten.

Zum Gegenstand der vorliegenden Erfindung gehört auch, wie eingangs erwähnt, ein Haarfärbemittel, bei dem die Zugabe eines Oxidationsmittels erforderlich ist. Es enthält außer den Farbstoffen gemäß der angegebenen Formel (I) und gegebenenfalls bekannten, direkt auf das Haar aufziehenden Farbstoffen noch zusätzliche, bekannte Oxidationsfarbstoffe, die einer oxidativen Entwicklung bedürfen.

Bei diesen Oxidationsfarbstoffen handelt es sich hauptsächlich um aromatische p-Diamine und p-Aminophenole, wie beispielsweise p-Toluylendiamin, p-Phenylendiamin und p-Aminophenol, welche zum Zwecke der Nuancierung der Färbungen mit sogenannten Modifiern, wie zum Beispiel m-Phenylendiamin, Resorcin, m-Aminophenol und anderen, kombiniert werden.

Derartige zu Haarfärbung bekannte und übliche Oxidationsfarbstoffe sind unter anderem in dem Buch von E. Sagarin, "Cosmetics", Science and Technology (1957), Interscience Publishers Inc., New York, Seiten 503 ff. sowie in dem Buch von H. Janistyn, "Handbuch der Kosmetika und Riechstoffe" (1973), Seiten 338 ff., beschrieben.

Mit Mischungen aus diesen Oxidationsfarbstoffen und den Farbstoffen gemäß der angegebenen Formel (I) lassen sich sehr gut natürliche Blond- und Brauntöne, aber auch modische Nuancen herstellen.

Die Farbstoffe gemäß der Formel (I) sind in diesem Färbemittel mit Oxidationsmittel-Zugabe in einer Konzentration von etwa 0,01 bis 2,0 Gewichtsprozent, vorzugsweise 0,01 bis 1,0 Gewichtsprozent, enthalten. Der Gesamtgehalt an Farbstoffen in diesem Färbemittel beträgt etwa 0,1 bis 5,0 Gewichtsprozent.

Oxidationshaarfärbemittel sind im allgemeinen alkalisch eingestellt, vorzugsweise auf pH-Werte von etwa 8,0 bis 11,5, wobei die Einstellung insbesondere mit Ammoniak erfolgt. Es können dazu aber auch andere organische Amine, zum Beispiel Monoethanolamin oder Triethanolamin, Verwendung finden. Als Oxidationsmittel zur Entwicklung der Haarfärbungen kommen hauptsächlich Hydrogenperoxid und dessen Additionsverbindungen in Betracht. Die Zubereitungsform dieses Haarfärbemittels kann die gleiche wie bei dem Haarfärbemittel ohne Oxidationsmittel-Zugabe sein. Vorzugsweise ist sie die einer Creme oder eines Gels.

Übliche Zusätze in Cremes, Emulsionen oder Gelen sind zum Beispiel Lösungsmittel wie Wasser, niedere aliphatische Alkohole, beispielsweise Ethanol, Propanol, Isopropanol, Glycerin oder Glykole, wie zum Beispiel Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen, wie zum Beispiel Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker, wie zum Beispiel höhere Fettalkohole, Bentonit, Stärke, Polyacrylsäure, Cellulosederivate wie zum Beispiel Carboxymethylcellulose, Alginate, Vaseline, Paraffinöl und Fettsäuren sowie außerdem Pflegestoffe, wie zum Beispiel Lanolinderivate, Cholesterin, Pantothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, während die Verdicker in einer Menge von etwa 0,1 bis 25 Gewichtsprozent und die Pflegestoffe in einer Menge von etwa 0,1 bis 5 Gewichtsprozent in den Zubereitungen enthalten sein können.

Die Anwendung der genannten Zubereitungen, bei denen die Zugabe eines Oxidationsmittels erforderlich ist, erfolgt in bekannter Weise, indem man die Haarfärbemittel vor der Behandlung mit dem Oxidationsmittel vermischt und eine zur Färbung des Haares ausreichende Menge der Mischung, im allgemeinen etwa 50 bis 150 ml, auf das Haar aufträgt. Nach einer für die Haarfärbung ausreichenden Einwirkungszeit, welche üblicherweise etwa 10 bis 45 Minuten beträgt, wird das Haar mit Wasser, gegebenenfalls anschließend mit der wäßrigen Lösung einer schwachen organischen Säure, wie zum Beispiel Zitronensäure oder Weinsäure, gespült und sodann getrocknet.

Hinsichtlich der färberischen Möglichkeiten bieten die erfindungsgemäßen Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt. Hierbei werden die Färbemittel je nach Zusammensetzung entweder in Verbindung mit Hydrogenperoxid oder auch ohne Oxidationsmittel angewandt.

Die 2,6-Dinitro-anilinderivate der Formel (I) sind zum Teil bekannt. So ist zum Beispiel für folgende Verbindungen die Herstellung in der Literatur beschrieben:

| Substituenten X | Substituenten R | Literatur |
|---|---|---|
| $CH_3$ | H | W.Staedel, Liebigs Ann. Chem. **217,** 186 (1883) |
| $CH_3$ | $CH_3$ | L.Gattermann, Ber. Dtsch. Chem. Ges. **18,** 1487 (1885) |
| $CH_3$ | $C_2H_4OH$ | DE-OS 32 00 787 |
| $CH_3$ | $CH_2CH(OH)CH_2OH$ | DE-OS 32 00 787 |
| $CH_3$ | $C_2H_5$ | L.Gattermann, Ber. Dtsch. Chem. Ges. **18,** 1485 (1885) |
| $CH_3$ | $C_3H_7$ | A.Hantzsch, Ber. Dtsch. Chem. Ges. **43,** 1673 (1910) |
| $CH_3$ | $C_4H_9$ | J.Reilly und W.J.Hickinbottom J.Chem.Soc.(London) **113,** 990(1918) |
| $CF_3$ | H | C.M.Jagupolskii u. V.S.Mospan, Ukr.Chim.Z. **21,** 81, 83 (1955), C.A. **49,** 8866 (1955) |

| | | |
|---|---|---|
| $CF_3$ | $C_2H_4OH$ | B.F.Malichenko, E.M.Levchenko und L.M. Yagupolskii, Ukr. Chim. Z. **33**, 717-719(1967), C.A. **68**, 39228, (1968) |
| Cl | H | R.C.Elderfield, W.J.Gensler und O.Birstein, J.Org.Chem. **11**,812,820 (1946) |
| Cl | $C_2H_4OH$ | K.F.Waldkötter, Recueil Trav. Chimiques Pays-Bas **57**, 1294, |
| Br | $C_2H_4OH$ | 1299, 1301 (1938) |
| Cl | $C_2H_5$ | K.F.Waldkötter, Recueil Trav. Chimiques Pays-Bas **58**, 132, |
| Br | $C_2H_5$ | 136 (1939) |
| Br | H | G.S.Hammond, F.J.Modic und R.M.Hedges, J.Am.Chem.Soc. **75**, 1388, 1389 (1953) |
| $OCH_3$ | H | F.Reverdin, Ber. Dtsch. Chem. Ges. **42**, 1524 (1909) |
| $OC_2H_5$ | H | F.Reverdin, Helv. Chimica Acta **12**, 117, 119, 1058 (1929) |
| $OC_2H_5$ | $CH_3$ | F.Reverdin und F.Liebl, J.prakt.Chemie 2 **86**, 205 (1912) |
| $OCH_3$ | $CH_3$ | H.H.Hodgson und J.H.Crook, J.Chem.Soc.(London) **1933**, 825, 827 |

Gegenstand der vorliegenden Erfindung sind weiterhin neue 2,6-Dinitro-anilinderivate der allgemeinen Formel (II)

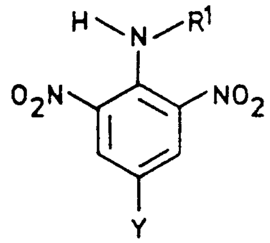

(II),

worin $R^1$ die Bedeutung $C_2$-$C_4$-Monahydroxyalkyl oder $C_3$-$C_4$-Dihydroxyalkyl hat und Y einen der Reste $C_2$-$C_4$-Monohydroxyalkyl, $C_1$-$C_4$-Perfluoralkyl, $C_1$-$C_4$-Alkoxy, $C_2$-$C_4$-Monohydroxyalkoxy, $C_3$-$C_4$-Dihydroxy-alkoxy oder Halogen darstellt, unter der Voraussetzung, daß, wenn Y Chlor oder Brom ist, $R^1$ nicht Hydroxyethyl bedeutet und wenn Y Trifluormethyl ist, $R^1$ nicht Monohydroxyalkyl bedeutet.

Beispiele für neue 2,6-Dinitro-anilinderivate der Formel (II) sind
2,6-Dinitro-4-trifluormethyl-(2',3'-dihydroxypropyl)-anilin,
2,6-Dinitro-4-methoxy-(2',3'-dihydroxypropyl)-anilin,
2,6-Dinitro-4-methoxy-(2'-hydroxyethyl)-anilin,
2,6-Dinitro-4-(2'-hydroxyethyloxy)-(2"'-hydroxyethyl)-anilin,
2,6-Dinitro-4-(2'-hydroxyethyloxy)-(2",3"-dihydroxypropyl)-anilin und
4-Chlor-2,6-dinitro-(2',3'-dihydroxypropyl)-anilin.

Die Herstellung der neuen Verbindungen der Formel (II) erfolgt durch nucleophilen Austausch einer Alkoxygruppe oder eines Halogenatoms (A) in der entsprechenden, mit Y substituierten Verbindung (III) gemäß der nachfolgenden Reaktionsgleichung.

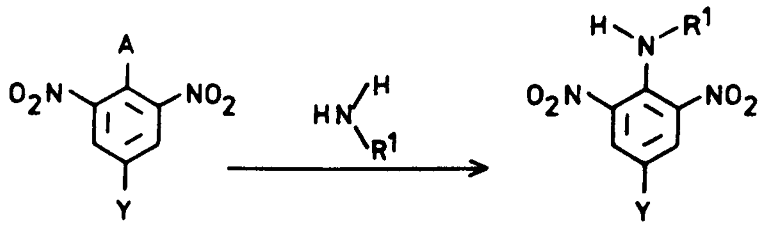

(III) (II)

Die Herstellung der 2-Nitro-anilinderivate der Formeln (I) und (II) ist ferner möglich durch Dinitrierung der gegebenenfalls, beispielsweise durch einen para-Toluolsulfonylrest, geschützten, in para-Stellung mit X oder Y substituierten Anilinderivate. Die 4-Chlor-oder 4-Brom-2,6-dinitro-anilinderivate können durch Chlorierung oder Bromierung des 2,6-Dinitro-anilins erhalten werden. Weiterhin lassen sich die jeweiligen unsubstituierten Aminoverbindungen durch die üblichen Alkylierungsverfahren zu den N-Alkylverbindungen umsetzen.

Die nachfolgenden Beispiele sollen den Gegenstand der Anmeldung weiter erläutern.

**Herstellungsbeispiele**

**Beispiel 1:** 2,6-Dinitro-4-trifluormethyl-(2′,3′-dihydroxypropyl)-anilin

1 g 4-Chlor-3,5-dinitro-benzotrifluorid wird in 4 ml Dimethylsulfoxid gelöst. Unter Rühren läßt man 1 ml 2,3-Dihydroxy-propylamin zutropfen. Nach dem Abklingen der exothermen Reaktion rührt man das Gemisch 1/2 Stunde lang bei Raumtemperatur und gießt es anschließend auf Eis. Von den ausgefallenen Kristallen wird abgesaugt. Nach dem Trocknen erhält man 1,0 g (83 Prozent der Theorie) des gelben Farbstoffes mit einem Schmelzpunkt von 129 - 130 Grad Celsius.

Massenspektrum:

325 ($M^+$, 14), 264 (100), 247 (10), 217 (16), 160 (16), 159 (12), 61 (18).

Für dieses und alle folgenden Massenspektren gilt:
Alle Peaks, deren Intensität größer als 10 Prozent beträgt, werden in m/e (relative Intensität in Prozent) angegeben.

UV-Spektrum:

208 (4,18), 235 sh (3,99), 310 (3,67), 350 (3,70), 416 (3,39)

Für dieses und alle folgenden UV-Spektren gilt:

Lösungsmittel: Ethanol

Die Peaks werden durch die Angabe der Wellenlänge des Maximums in Nanometer (logarithmischer Extinktionskoeffizient) charakterisiert.

**Beispiel 2:** 2,6-Dinitro-4-methoxy-(2'-hydroxyethyl)-anilin

1 g 2,6-Dinitro-hydrochinondimethylether (B. Reichert und W. Turkewitsch, Arch. der Pharmazie 276, 397, 406 (1938)) wird in 15 ml Ethanolamin 1 Stunde bei Raumtemperatur gerührt. Anschließend wird der Ansatz auf Eis gegossen und mit Essigsäure neutralisiert. Von den ausgefallenen roten Kristallen wird abgesaugt. Man erhält 0,9 g (78 Prozent der Theorie) des Produkts mit einem Schmelzpunkt von 104 Grad Celsius.

$^1H$ NMR-Spektrum:

8,1 (breit, OH, NH, das Signal verschwindet beim Schütteln der Probe mit $D_2O$), 7,78 (s, 3-H, 5-H), 3,85 (s, $OCH_3$), 3,84 (t, J = 5 Hz, HN-$CH_2CH_2$-OH), 3,13 (q, J = 5 Hz, HN-$C\underline{H}_2$-, das Signal bildet beim Schütteln der Probe mit $D_2O$ ein t, J = 5 Hz), 1,68 (breit, OH, NH, das Signal verschwindet beim Schütteln der Probe mit $D_2O$)

Für dieses und alle folgenden NMR-Spektren gilt:
Die Angaben der chemischen Verschiebung erfolgt in delta (ppm), die der Kopplungskonstanten in Hertz.
Standard: Tetramethylsilan
Lösungsmittel: $CDCl_3$, wenn nicht anders angegeben s = Singulett, d = Duplett, t = Triplett, q = Quartett, m = Multiplett

**Beispiel 3:** 2,6-Dinitro-4-methoxy-(2',3'-dihydroxypropyl)-anilin

1 g 2,6-Dinitro-hydrochinondimethylether (siehe Beispiel 2) wird in 8 ml Dimethylsulfoxid gelöst und unter Rühren mit 3,6 g 1-Amino-propandiol-2,3 versetzt. Man rührt 3 1/2 Stunden lang bei Raumtemperatur, gießt anschließend den Ansatz auf Eis und saugt von den roten Kristallen ab. Man erhält 0,95 g (74 Prozent der Theorie) des gesuchten Farbstoffes.

NMR-Spektrum (in DMSO-$d_6$):

8,02 (t, J = 5 Hz, NH; das Signal verschwindet beim Schütteln der Probe mit $D_2O$), 7,89 (s, 3-H, 5-H), 5,13 (q, J = 5 Hz, OH; das Signal verschwindet beim Schütteln der Probe mit $D_2O$), 4,65 (t, J = 5 Hz, OH; das Signal verschwindet beim Schütteln der Probe mit $D_2O$), 3,82 (s, $OCH_3$),

$$3{,}59\ (m,\ CH_2\overset{\overset{OH}{|}}{C}H-C\underline{H}_2OH),$$

3,28 (m, -$C\underline{H}_2$OH), 2,88 (m, HN-$C\underline{H}_2$-)

UV-Spektrum:

234 (4,37), 468 (3,74)

**Beispiel 4:** 4-Chlor-2,6-dinitro-(2',3'-dihydroxypropyl)-anilin

0,25 g 4-Chlor-2,6-dinitro-anisol (Kohn und Kramer, Monatsh. Chemie 49, 154) werden bei 5 - 10 Grad Celsius in 2 ml 2,3-Dihydroxy-propylamin gelöst und eine halbe Stunde lang bei dieser Temperatur gerührt. Anschließend wird die orange Lösung auf Eis gegossen und man saugt von den ausgefallenen gelben Kristallen ab. Man erhält 0,2 g (64 Prozent der Theorie) des Farbstoffes mit einem Schmelzpunkt von 82 Grad Celsius.

| Elementaranalyse ($C_9H_{10}ClN_2O_5$): | | |
|---|---|---|
| | berechnet: | gefunden: |
| C % | 37,06 | 36,92 |
| H % | 3,45 | 3,49 |
| N % | 14,40 | 14,21 |

**Haarfärbebeispiele**

**Beispiel 5** Flüssiges Haarfärbemittel

| | |
|---|---|
| 0,30 g | 2,6-Dinitro-4-methyl-(2',3'-dihydroxypropyl)-anilin |
| 2,00 g | Laurylalkohol-diglykolethersulfat-Natriumsalz (28prozentige, wäßrige Lösung) |
| 2,00 g | Ammoniak, 25prozentig |
| 95,70 g | Wasser |
| 100,00 g | |

Gebleichtes Naturhaar wird 20 Minuten lang bei Raumtemperatur mit der Lösung nach Beispiel 5 behandelt, danach mit Wasser gespült und anschließend getrocknet. Das Haar ist leuchtend orange gefärbt.

**Beispiel 6** Farbfestiger

| | |
|---|---|
| 0,10 g | 2,6-Dinitro-4-trifluormethyl-(2',3'-dihydroxypropyl)-anilin |
| 2,00 g | Polyvinylpyrrolidon |
| 0,10 g | Glycerin |
| 40,00 g | Isopropanol |
| 57,80 g | Wasser |
| 100,00 g | |

Weiße menschliche Haare werden mit der Farbfestigerlösung eingelegt und getrocknet. Das Haar ist leuchtend zitronengelb gefärbt und gefestigt.

**Beispiel 7** Oxidationshaarfärbemittel in Cremeform

| | |
|---|---|
| 0,10 g | 2,6-Dinitro-4-(2'-hydroxyethoxy)-(2",3"-dihydroxypropyl)-anilin |
| 0,20 g | p-Phenylendiamin |
| 0,15 g | Resorcin |
| 0,03 g | m-Aminophenol |
| 15,00 g | Cetylalkohol |
| 3,50 g | Laurylalkohol-diglykolethersulfat-Natriumsalz (28prozentige, wäßrige Lösung) |
| 6,00 g | Ammoniak, 25prozentig |
| 75,02 g | Wasser |
| 100,00 g | |

50 g des vorstehenden Haarfärbemittels werden kurz vor der Anwendung mit 50 ml Wasserstoffperoxidlösung (6prozentig) gemischt. Das Gemisch wird anschließend auf graue menschliche Haare aufgetragen und 30 Minuten lang bei einer Temperatur von 40 Grad Celsius einwirken gelassen. Nach dem Spülen des Haares mit Wasser und dem anschließenden Trocknen hat es einen modischen, rötlichen Blondton angenommen.

**Beispiel 8** Haarfärbemittel in Cremeform

| | |
|---|---|
| 0,050 g | 4-Chlor-2,6-dinitro-(2',3'-dihydroxypropyl)-anilin |
| 0,250 g | 4-Bis(2'-hydroxyethyl)amino-1-methylamino-2-nitro-benzol |
| 0,020 g | 1-Amino-2-nitro-4-bis(2'-hydroxyethyl)amino-benzol |
| 0,025 g | Disperse Blue 23 (C.I. 61 545) |
| 7,500 g | Cetylalkohol |
| 1,750 g | Laurylalkohol-diglykolethersulfat-Natriumsalz (28prozentige, wäßrige Lösung) |
| 0,100 g | p-Hydroxybenzoesäuremethylester |
| 0,200 g | Ammoniak, 25 prozentige |
| 90,105 g | Wasser |
| 100,000 g | |

50 g des vorstehenden Haarfärbemittels werden auf weiße menschliche Haare aufgetragen und nach einer Einwirkungszeit von 20 Minuten mit Wasser ausgespült. Das Haar wird anschließend getrocknet. Es ist in einem natürlichen, braunen Farbton gefärbt.

**Beispiel 9** Flüssiges Haarfärbemittel

| | |
|---|---|
| 0,25 g | 2,6-Dinitro-4-methoxy-(2',3'-dihydroxypropyl)-anilin |
| 0,10 g | 1-Amino-4-(2',3'-dihydroxypropyl)-amino-2-nitro-5-chlor-benzol |
| 0,20 g | 4-Bis(2'-hydroxyethyl)amino-1-methylamino-2-nitro-benzol |
| 0,50 g | Hydroxyethylcellulose |
| 5,00 g | Laurylalkohol-diglykolethersulfat-Natriumsalz (28prozentige, wäßrige Lösung) |
| 10,00 g | Isopropanol |
| 10,00 g | Ammoniak, 25prozentig |
| 73,95 g | Wasser |
| 100,00 g | |

Gebleichtes Naturhaar wird 20 Minuten lang bei Raumtemperatur mit der Lösung nach Beispiel 9 behandelt. Nach der Spülung des Haares mit Wasser und der anschließenden Trocknung ist das Haar in einem modischen dunklen Beaujolaiston eingefärbt.

**Beispiel 10** Flüssiges Haarfärbemittel

| | |
|---|---|
| 0,30 g | 2,6-Dinitro-4-(2'-hydroxyethyloxy)-(2"-hydroxyethyl)-anilin |
| 0,05 g | 1,4-Bis(2'-hydroxyethyl)amino-2-nitro-benzol |
| 0,50 g | Hydroxyethylcellulose |
| 5,00 g | Laurylalkohol-diglykolethersulfat-Natriumsalz (28 prozentige, wäßrige Lösung) |
| 10,00 g | Isopropanol |
| 10,00 g | Ammoniak, 25prozentig |
| 74,15 g | Wasser |
| 100,00 g | |

Das vorstehende Färbemittel wird auf gebleichte Naturhaare 30 Minuten lang bei 40 Grad Celsius einwirken gelassen. Nach der Spülung des Haares mit Wasser und der anschließenden Trocknung ist das Haar in einem modischen Blondton gefärbt.

## Patentansprüche

1. Verwendung eines 2,6-Dinitro-anilinderivats der allgemeinen Formel (I)

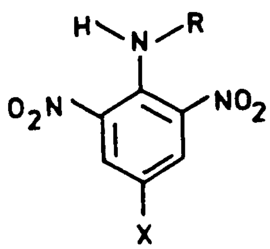

(I),

wobei R die Bedeutung H, $C_1$-$C_4$-Alkyl, $C_2$-$C_4$-Monohydroxyalkyl oder $C_3$-$C_4$-Dihydroxyalkyl hat, und X einen der Reste $C_1$-$C_4$-Alkyl, $C_2$-$C_4$-Monohydroxyalkyl, $C_1$-$C_4$-Perfluoralkyl, $C_1$-$C_4$-Alkoxy, $C_2$-$C_4$-Monohydroxyalkoxy, $C_3$-$C_4$-Dihydroxyalkoxy oder Halogen darstellt, als Farbstoff in Haarfärbemitteln.

2. Verwendung eines 2,6-Dinitro-anilinderivats der allgemeinen Formel (I), wobei R die Bedeutung 2-Hydroxyethyl oder 2,3-Dihydroxypropyl hat, und X einen der Reste $CH_3$, $CF_3$, $CH_2OH$, $OCH_3$, $OCH_2CH_2OH$, $OCH_2CH(OH)CH_2OH$, Cl oder Br darstellt, als Farbstoff in Haarfärbemitteln.

3. Mittel zur Färbung von Haaren mit einem Farbstoffgehalt und für Haarfärbemittel üblichen Zusätzen, dadurch gekennzeichnet, daß es 0,01 bis 2,0 Gewichtsprozent eines 2,6-Dinitro-anilinderivats der Formel (I) enthält.

4. Mittel nach Anspruch 3, dadurch gekennzeichnet, daß das 2,6-Dinitro-anilinderivat der Formel (I) ausgewählt ist aus 2,6-Dinitro-4-methyl-(2'-hydroxyethyl)-anilin, 2,6-Dinitro-4-methyl-(2',3'-dihydroxypropyl)-anilin, 2,6-Dinitro-4-trifluormethyl-(2',3'-dihydroxypropyl)-anilin, 2,6-Dinitro-4-trifluormethyl-(2'-hydroxyethyl)-anilin, 2,6-Dinitro-4-methoxy-(2',3'-dihydroxypropyl)-anilin, 2,6-Dinitro-4-methoxy-(2'-hydroxyethyl)-anilin, 2,6-Dinitro-4-(2'-hydroxyethyloxy)-(2"-hydroxyethyl)-anilin, 2,6-Dinitro-4-(2'-hydroxyethyloxy)-(2",3"-dihydroxypropyl)-anilin und 4-Chlor-2,6-dinitro-(2',3'-dihydroxypropyl)-anilin.

5. Mittel nach einem der Ansprüche 3 oder 4, dadurch gekennzeichnet, daß es mindestens einen der bekannten direkt auf das Haar aufziehenden Farbstoffe enthält.

6. Mittel nach Anspruch 5, dadurch gekennzeichnet, daß die bekannten direkt auf das Haar aufziehenden Farbstoffe ausgewählt sind aus 2-Amino-4-nitro-phenol, Pikraminsäure, 2-(2'-Hydroxyethyl)amino-4,6-dinitro-phenol, 1-(2'-Hydroxyethyl)amino-2-amino-4-nitro-benzol, 4-(2'-Ureidoethyl)amino-nitrobenzol, 4-(2',3'-Dihydroxypropyl)amino-3-nitro-trifluormethylbenzol, 4-(2'-Hydroxyethyl)amino-3-nitro-toluol, 2-Nitro-4-(2'-hydroxyethyl)amino-anilin, 1,4-Bis(2'-hydroxyethyl)amino-4-N-ethyl-2-nitro-benzol, 2,5-Bis(2'-hydroxyethyl)amino-nitrobenzol, 1-Amino-4-(2',3'-dihydroxypropyl)amino-2-nitro-5-chlor-benzol, 4-Bis(2'-hydroxyethyl)amino-1-methyl-amino-2-nitro-benzol, 1-Amino-2-nitro-4-bis(2'-hydroxyethyl)amino-benzol,

Basic Violet 1 (C.I. 42 535), Acid Brown 4 (C.I. 14 805), Disperse Violet 4 (C.I. 61 105), Disperse Blue 23 (C.I. 61 545), 1,4,5,8-Tetraamino-anthrachinon und 1,4-Diamino-anthrachinon.

**7.** Mittel nach einem der Ansprüche 5 oder 6, dadurch gekennzeichnet, daß es in Form eines Haarfärbemittels mit zusätzlicher Haarfestigung vorliegt und mindestens ein in der Kosmetik üblicherweise verwendetes Polymerisat oder natürliches Polymer enthält.

**8.** Mittel nach Anspruch 7, dadurch gekennzeichnet, daß das Polymerisat oder das natürliche Polymer ausgewählt ist aus Chitosan, Polyvinylpyrrolidon, Polyvinylacetat, Polyvinylalkohol, Polyacrylsäure, Polymethacrylsäure, Polyacrylnitril, Polyvinyllactam oder Copolymerisaten aus diesen Verbindungen.

**9.** Mittel nach einem der Ansprüche 3 oder 4, dadurch gekennzeichnet, daß es mindestens einen der bekannten Oxidationshaarfarbstoffe enthält.

**10.** Mittel nach Anspruch 9, dadurch gekennzeichnet, daß der bekannte Oxidationshaarfarbstoff ausgewählt ist aus p-Toluylendiamin, p-Phenylendiamin, p-Aminophenol, m-Phenylendiamin, Resorcin und m-Aminophenol.

**11.** 2,6-Dinitro-anilinderivat der allgemeinen Formel (II)

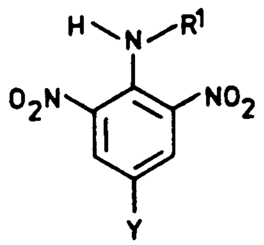

(II),

worin $R^1$ die Bedeutung $C_2$-$C_4$-Monohydroxyalkyl oder $C_3$-$C_4$-Dihydroxyalkyl hat und Y einen der Reste $C_2$-$C_4$-Monohydroxyalkyl, $C_1$-$C_4$-Perfluoralkyl, $C_1$-$C_4$-Alkoxy, $C_2$-$C_4$-Monohydroxyalkoxy, $C_3$-$C_4$-Dihydroxyalkoxy oder Halogen darstellt, unter der Voraussetzung, daß, wenn Y Chlor oder Brom ist, $R^1$ nicht Hydroxyethyl bedeutet und wenn Y Trifluormethyl ist, $R^1$ nicht Monohydroxyalkyl bedeutet.

**12.** 2,6-Dinitro-4-trifluormethyl-(2',3'-dihydroxypropyl)-anilin

**13.** 2,6-Dinitro-4-methoxy-(2',3'-dihydroxypropyl)-anilin

**14.** 2,6-Dinitro-4-methoxy-(2'-hydroxyethyl)-anilin

**15.** 2,6-Dinitro-4-(2'-hydroxyethyloxy)-(2"-hydroxyethyl)-anilin

**16.** 2,6-Dinitro-4-(2'-hydroxyethyloxy)-(2",3"-dihydroxypropyl)-anilin

**17.** 4-Chlor-2,6-dinitro-(2',3'-dihydroxypropyl)-anilin

## Claims

**1.** Use of a 2,6-dinitro-aniline derivative of the general formula (I)

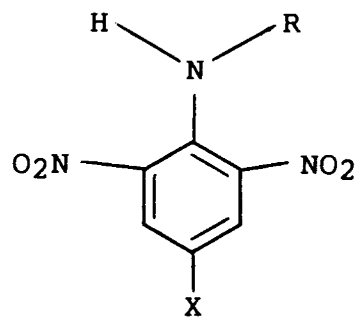

(I),

wherein R means H, $C_1$-$C_4$-alkyl, $C_2$-$C_4$-monohydroxyalkyl or $C_3$-$C_4$-dihydroxyalkyl, and X represents one of the residues $C_1$-$C_4$-alkyl, $C_2$-$C_4$-monohydroxyalkyl, $C_1$-$C_4$-perfluoroalkyl, $C_1$-$C_4$-alkoxy, $C_2$-$C_4$-monohydroxyalkoxy, $C_3$-$C_4$-dihydroxyalkoxy or halogen, as dye in hair colouring compositions.

**2.** Use of a 2,6-dinitro-aniline derivative of the general formula (I), wherein R means 2-hydroxyethyl or 2,3-dihydroxypropyl, and X represents one of the residues $CH_3$, $CF_3$, $CH_2OH$, $OCH_3$, $OCH_2CH_2OH$, $OCH_2CH(OH)CH_2OH$, Cl or Br as dye in hair colouring compositions.

**3.** Composition for dyeing hair with a dye content and conventional additives for hair colouring compositions, characterised in that it contains 0.01 to 2.0 percent by weight of a 2,6-dinitro-aniline derivative of the formula (I).

**4.** Composition according to Claim 3, characterised in that the 2,6-dinitro-aniline derivative of the formula (I) is selected from 2,6-dinitro-4-methyl-(2'-hydroxyethyl)-aniline, 2,6-dinitro-4-methyl-(2'-,3'-dihydroxypropyl)-aniline, 2,6-dinitro-4-trifluoromethyl-(2',3'-dihydroxypropyl)-aniline, 2,6-dinitro-4-trifluoromethyl-(2'-hydroxyethyl)-aniline, 2,6-dinitro-4-methoxy-(2',3'-dihydroxypropyl)-aniline, 2,6-dinitro-4-methoxy-(2'-hydroxyethyl)-aniline, 2,6-dinitro-4-(2'-hydroxyethyloxy)-(2''-hydroxyethyl)-aniline, 2,6-dinitro-4-(2'-hydroxyethyloxy)-(2'',3''-dihydroxypropyl)-aniline and 4-chloro-2,6-dinitro-(2',3'-dihydroxypropyl)-aniline.

**5.** Composition according to either one of the Claims 3 or 4, characterised in that it contains at least one of the known direct dyes for hair.

**6.** Composition according to Claim 5, characterised in that the known direct dyes for hair are selected from 2-amino-4-nitrophenol, picramic acid, 2-(2'-hydroxyethyl)amino-4,6-dinitro-phenol, 1(2'-hydroxyethyl)amino-2-amino-4-nitro-benzene, 4-(2'-ureidoethyl)amino-nitrobenzene, 4-(2',3'-dihydroxypropyl)-amino-3-nitro-trifluoromethylbenzene, 4-(2'-hydroxyethyl)amino-3-nitrotoluene, 2-nitro-4-(2'-hydroxyethyl)amino-aniline, 1,4-bis(2'-hydroxyethyl)amino-4-N-ethyl-2-nitro-benzene, 2,5-bis(2'-hydroxyethyl)-amino-nitrobenzene, 1-amino-4-(2',3'-dihydroxypropyl)amino-2-nitro-5-chlorobenzene, 4-bis(2'-hydroxyethyl)amino-1-methylamino-2-nitro-benzene, 1-amino-2-nitro-4-bis(2'-hydroxyethyl)-aminobenzene, Basic Violet 1 (C.I. 42 535), Acid Brown 4 (C.I. 14 805), Disperse Violet 4 (C.I. 61 105), Disperse Blue 23 (C.I. 61 545), 1,4,5,8-tetraamino-anthraquinone and 1,4-diamino-anthraquinone.

**7.** Composition according to either one of the Claims 5 or 6, characterised in that it is in the form of a hair colouring medium with additional hair strengthener and in that it contains at least one polymerisation product or natural polymer usually used in beauty culture.

**8.** Composition according to Claim 7, characterised in that the polymerisation product or the natural polymer is selected from chitosan, polyvinylpyyrolidone, polyvinylacetate, polyvinylalcohol, polyacrylic acid, polymethacrylic acid, polyacarylonitrile, polyvinyllactam or copolymers of these compounds.

**9.** Composition according to either Claims 3 or 4, characterised in that it contains at least one of the known oxidation hair colouring agents.

**10.** Composition according to Claim 9, characterised in that the known oxidation hair colouring agent is selected from p-toluylendiamine, p-phenylendiamine, p-aminophenol, m-phenylendiamine, resorcinol

and m-aminophenol.

**11.** 2,6-dinitro-aniline derivative of the general formula (II)

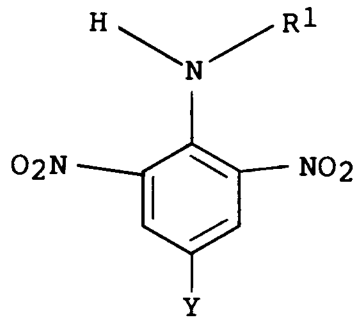

(II),

in which $R^1$ means $C_2$-$C_4$-monohydroxyalkyl or $C_3$-$C_4$-dihydroxyalkyl and Y represents one of the residues $C_2$-$C_4$-monohydroxyalkyl, $C_1$-$C_4$-perfluoralkyl, $C_1$-$C_4$-alkoxy, $C_2$-$C_4$-monohydroxyalkoxy, $C_3$-$C_4$-dihydroxyalkoxy or halogen, provided that if Y is chlorine or bromine, $R^1$ is not hydroxyethyl and if Y is trifluoromethyl, $R^1$ is not monohydroxyalkyl.

**12.** 2,6-dinitro-4-trifluoromethyl-(2',3'-dihydroxypropyl)-aniline.

**13.** 2,6-dinitro-4-methoxy-(2',3'-dihydroxypropyl)-aniline.

**14.** 2,6-dinitro-4-methoxy-(2'-hydroxyethyl)-aniline.

**15.** 2,6-dinitro-4-(2'-hydroxyethyloxy)-(2''-hydroxyethyl)-aniline.

**16.** 2,6-dinitro-4-(2'-hydroxyethyloxy)-(2' ,3' -dihydroxypropyl)-aniline.

**17.** 4-chloro-2,6-dinitro-(2',3'-dihydroxypropyl)-aniline.

## Revendications

**1.** Utilisation d'un dérivé de 2,6-dinitro-aniline répondant à la formule générale (I)

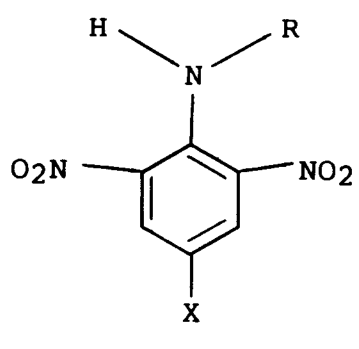

(I),

dans laquelle R représente H, un alkyle en $C_1$ à $C_4$, une monohydroxyalkyle en $C_2$ à $C_4$, et X représente l'un des restes alkyle en $C_1$ à $C_4$, monohydroxyalkyle en $C_2$ à $C_4$, perfluoralkyle en $C_1$ à $C_4$, alkoxy en $C_1$ à $C_4$, monohydroxyalkoxy en $C_2$ à $C_4$, dihydroxyalkoxy en $C_3$ à $C_4$ ou halogène, comme colorant dans des produits de coloration des cheveux.

**2.** Utilisation d'un dérivé de 2,6-dinitro-aniline répondant à la formule générale (I) où R représente le 2-hydroxyéthyle ou le 2,3-dihydroxypropyle, et X représente l'un des restes $CH_3$, $CF_3$, $CH_2OH$, $OCH_3$, $OCH_2CH_2OH$, $OCH_2CH(OH)CH_2OH$, Cl ou Br, comme colorants dans des produits de coloration des cheveux.

3. Produit de coloration des cheveux renfermant un colorant et des additifs usuels pour les produits de coloration des cheveux, caractérisé en ce qu'il renferme 0,01 à 2,0 % en poids d'un dérivé de 2,6-dinitro-aniline de la formule (I).

4. Produit selon la revendication 3, caractérisé en ce que le dérivé de 2,6-dinitro-aniline de la formule (I) est choisi parmi la 2,6-dinitro-4-méthyl-(2'-hydroxyéthyl)-aniline,la 2,6-dinitro-4-méthyl-(2',3'-dihydroxypropyl)-aniline, la 2,6-dinitro-4-trifluorométhyl-(2',3'-dihydroxypropyl)-aniline, la 2,6-dinitro-4-trifluorométhyl-(2'-hydroxyéthyl)-aniline, la 2,6-dinitro-4-méthoxy-(2',3'-dihydroxypropyl)-aniline, la 2,6-dinitro-4-méthoxy-(2'-hydroxyéthyl)-aniline, la 2,6-dinitro-4-(2'-hydroxyéthyloxy)-(2''-hydroxyéthyl)-aniline, la 2,6-dinitro-4-(2'-hydroxyéthyloxy)-(2'',3''-dihydroxypropyl)-aniline et la 4-chloro-2,6-dinitro-(2',3'-dihydroxypropyl)-aniline.

5. Produit selon l'une des revendications 3 ou 4, caractérisé en ce qu'il renferme l'un au moins des colorants à montée directe sur les cheveux connus.

6. Produit selon la revendication 5, caractérisé en ce que les colorants à montée directe sur les cheveux sont choisis parmi le 2-amino-4-nitro-phénol, l'acide picraminique, le 2-(2'-hydroxyéthyl)amino-4,6-dinitro-phénol, le 1-(2'-hydroxyéthyl)amino-2-amino-4-nitro-benzène, le 4-(2'-uréidoéthyl)-aminonitrobenzène, le 4-(2',3'-dihydroxypropyl)amino-3-nitro-trifluorométhyl-benzène, le 4-(2'-hydroxyéthyl)amino-3-nitrotoluène, la 2-nitro-4-(2'-hydroxyéthyl)amino-aniline, le 1,4-bis(2'-hydroxyéthyl)-amino-4-N-éthyl-2-nitro-benzène, le 2,5-bis(2'-hydroxyéthyl)amino-nitrobenzène, le 1-amino-4-(2', 3'-dihydroxypropyl)amino-2-nitro-5-chlorobenzène, le 4-bis(2'-hydroxyéthyl)amino-1-méthylamino-2-nitro-benzène, le 1-amino-2-nitro-4bis(2'-hydroxyéthyl)amino-benzène, Basic Violet 1 (C.I. 42 535), Acid Brown 4 (C.I. 14 805), Disperse Violet 4 (C.I. 61 105), Disperse Blue 23 (C.I. 61 545), 1,4,5,8-tetraamino-anthraquinone et 1,4-diaminoanthraquinone.

7. Produit selon l'une des revendications 5 ou 6, caractérisé en ce qu'il se présente sous la forme d'un produit de coloration des cheveux avec fixation des cheveux supplémentaire et renferme au moins un polymérisat ou polymère naturel utilisé notamment en cosmétique.

8. Produit selon la revendication 7, caractérisé en ce que le polymérisat ou le polymére naturel est choise parmi le chitosane, la polyvinylpyrrolidone, l'acétate de polyvinyle, l'alcool polyvinylique, l'acide polyacarylique, l'acide polyméthacrylique, le polyacrylonitrile, le polyvinyllactame ou des copolymérisats de ces composés.

9. Produit selon l'une des revendications 3 ou 4, caractérisé en ce qu'il renferme l'un au moins des colorants par oxydation pour les cheveux connus.

10. Produit selon la revendication 9, caractérisé en ce que le colorant par oxydation pour les cheveux connu est choisi parmi la p-toluylène-diamine, la p-phénylène-diamine, le p-aminophénol, la m-phénylène-diamine, la résorcine, le m-aminophénol.

11. Dérivé de 2,6-dinitro-aniline répondant à la formule générale (II)

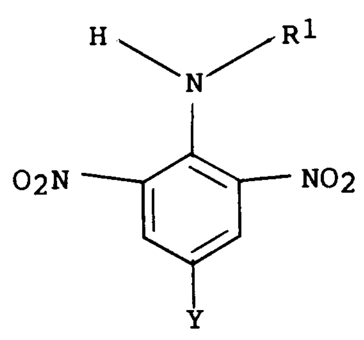

(II),

dans laquelle $R^1$ représente un monohydroxyalkyle en $C_2$ à $C_4$ ou un dihydroxy alkyle en $C_3$ à $C_4$ et Y

représente l'un des restes monohydroxyalkyle en $C_2$ à $C_2$, perfluoralkyle en $C_1$ à $C_4$, alkoxy en $C_1$ à $C_4$, monohydroxyalkoxy en $C_2$ à $C_4$, dihydroxyalkoxy en $C_3$ à $C_4$ ou halogène, à condition que, lorsque Y représente le chlore ou le brome, $R^1$ ne représente pas l'hydroxyéthyle et que, lorsque Y représente le trifluorométhyle, $R^1$ ne représente pas un monohydroxy-alkyle.

**12.** 2,6-dinitro-4-trifluorométhyl-(2',3'-dihydroxypropyl)-aniline.

**13.** 2,6-dinitro-4-méthoxy-(2',3'-dihydroxypropyl)-aniline.

**14.** 2,6-dinitro-4-méthoxy-(2'-hydroxyéthyl)-aniline.

**15.** 2,6-dinitro-4-(2'-hydroxyéthyloxy)-(2''-hydroxyéthyl)-aniline.

**16.** 2,6-dinitro-4-(2'-hydroxyéthyloxy)-(2'',3''-dihydroxypropyl)-aniline.

**17.** 4-chloro-2,6-dinitro-(2',3'-dihydroxypropyl)-aniline.